# EUROPÄISCHE PATENTSCHRIFT

(11) **EP 2 498 729 B1**
(45) Veröffentlichungstag und Bekanntmachung des Hinweises auf die Patenterteilung: **05.03.2014**
(21) Anmeldenummer: 10784689.1
(22) Anmeldetag: 12.11.2010
(51) Int. Cl.: A61F 2/68, A61F 2/64, A61F 2/66

(54) **VERFAHREN ZUR STEUERUNG EINES KÜNSTLICHEN ORTHETISCHEN ODER PROTHETISCHEN GELENKES**
METHOD FOR CONTROLLING AN ARTIFICIAL ORTHOTIC OR PROSTHETIC JOINT
PROCÉDÉ POUR COMMANDER UNE ARTICULATION ORTHÉTIQUE OU PROTHÉTIQUE ARTIFICIELLE

(30) Priorität: 13.11.2009 DE 102009052894
(43) Veröffentlichungstag der Anmeldung: 19.09.2012
(73) Patentinhaber: Otto Bock Healthcare Products GmbH, 1070 Wien (AT)
(72) Erfinder: SEYR, Martin, A-1040 Wien (AT); PLESA, Christian, A-2384 Breitenfurt (AT)
(74) Vertreter: Stornebel, Kai
(86) Internationale Anmeldenummer: PCT/EP2010/006894
(87) Internationale Veröffentlichungsnummer: WO 2011/057793

(56) Entgegenhaltungen:
- WO-A2-2007/110585
- DE-A1-102007 053 389
- US-A1- 2008 114 272

## Beschreibung

Die Erfindung betrifft ein Verfahren zur Steuerung eines künstlichen orthetischen oder prothetischen Gelenkes einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes über Sensoren Zustandsinformationen bereitgestellt werden.

Künstliche Gelenke, insbesondere Kniegelenke, für Orthesen oder Prothesen weisen ein oberes Anschlussteil und ein unteres Anschlussteil auf, die über eine Gelenkeinrichtung miteinander verbunden sind. An dem oberen Anschlussteil sind im Falle eines Kniegelenkes Aufnahmen für einen Oberschenkelstumpf oder eine Oberschenkelschiene angeordnet, während an dem unteren Anschlussteil ein Unterschenkelschaft oder eine Unterschenkelschiene angeordnet ist. Im einfachsten Fall ist das obere Anschlussteil mit dem unteren Anschlussteil durch ein einachsiges Gelenk verschwenkbar miteinander verbunden. Eine solche Anordnung ist nur in Ausnahmefällen ausreichend, um den gewünschten Erfolg, beispielsweise eine Unterstützung bei dem Einsatz einer Orthese oder ein natürliches Gangbild bei dem Einsatz in einer Prothese, zu gewährleisten.

Um die unterschiedlichen Anforderungen während der verschiedenen Phasen eines Schrittes oder bei anderen Verrichtungen möglichst natürlich darzustellen oder zu unterstützen, sind Widerstandseinrichtungen vorgesehen, die einen Flexionswiderstand oder einen Extensionswiderstand bereitstellen. Über den Flexionswiderstand wird eingestellt, wie leicht das untere Anschlussteil gegenüber dem oberen Anschlussteil in Flexionsrichtung verschwenkbar ist. Bei einem Kniegelenk wird daher über den Flexionswiderstand eingestellt, wie leicht der Unterschenkelschaft oder die Unterschenkelschiene im Verhältnis zu dem Oberschenkelschaft oder der Oberschenkelschiene bei einer aufgebrachten Kraft nach hinten schwingt. Der Extensionswiderstand bremst die Vorwärtsbewegung des Unterschenkelschaftes oder der Unterschenkelschiene und kann einen Streckanschlag ausbilden. Bei anderen Gelenktypen, wie dem Hüftgelenk oder dem Knöchelgelenk, gelten diese Ausführungen entsprechend den kinematischen Verhältnissen.

Über einstellbare Widerstandseinrichtungen ist es möglich, den jeweiligen Flexions- und/oder Extensionswiderstand an den Nutzer der prothetischen oder orthetischen Einrichtung anzupassen oder auf verschiedene Gang- oder Bewegungssituationen einzugehen, um bei sich verändernden Bedingungen einen angepassten Widerstand bereitstellen zu können.

Aus der DE 10 2008 008 284 A1 ist ein orthopädietechnisches Kniegelenk mit einem Oberteil und einem verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. Der Extensionsanschlag wird in Abhängigkeit von den ermittelten Sensordaten eingestellt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momentenarmes Anheben des Prothesenfußes detektiert und die Flexionsdämpfung in einer Anhebephase auf unterhalb eines Niveaus, das für eine Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2007 053 389 A1 beschreibt ein Verfahren und eine Vorrichtung zur Steuerung eines orthopädischen Gelenkes einer unteren Extremität mit zumindest einem Freiheitsgrad mit einem verstellbaren Aktuator zur Anpassung einer orthopädischen Einrichtung, die oberseitige Anschlussmittel an eine Gliedmaße und ein distal zu Anschlussmitteln gelenkig angeordnetes orthopädisches Gelenk aufweist, an Gehsituationen, die von einem Gehen in der Ebene abweichen. Dabei werden mehrere Parameter der orthopädischen Einrichtung über Sensoren erfasst, die erfassten Parameter mit Kriterien, die anhand mehrerer Parameter und/oder Parameterverläufe erstellt worden und in einer Rechnereinheit abgelegt sind, verglichen und ein Kriterium ausgewählt, das anhand der ermittelten Parameter oder Parameterverläufe geeignet ist. Auf der Grundlage des gewählten Kriteriums werden Beugungswiderstände, Bewegungsumfänge, Antriebskräfte und/oder deren Verläufe eingestellt, um Sonderfunktionen zu steuern, die vom Gehen in der Ebene abweichen. Ein Kippwinkel eines Teils der orthopädischen Einrichtung im Raum und/oder ein Verlauf einer Kippwinkeländerung eines Teils der orthopädischen Einrichtung können als Parameter herangezogen werden.

Weiterhin sind aus dem Stand der Technik so genannte Bremskniegelenke bekannt, bei denen mechanisch bei wachsender Axialbelastung der Flexions- und Extensionswiderstand erhöht wird. Dies wird im einfachsten Fall dadurch erreicht, dass zwei Bremsflächen vorgesehen sind, die durch eine Bodenreaktionskraft aufeinander gepresst werden. Eine solche Ausgestaltung ist an der Bremseinrichtung ist für moderne Prothesenkniegelenke mit gesteuerten Widerstandseinrichtungen nicht einsetzbar.

Es hat sich bewährt, dass Kniegelenke in der Standphase während des Gehens oder während des Stehens einen hohen Widerstand bieten, wobei das Gelenk nicht vollständig gesperrt wird. Bei einem vollständig gestreckten Kniegelenk wird das Beugen des Gelenks dadurch verhindert, dass der Kraftvektor vor der Gelenksachse liegt und somit das Gelenk in den Streckanschlag gedrückt wird. Sobald der Kraftvektor hinter die Gelenkachse wandert, besteht die Gefahr, dass das Gelenk einknickt. Daher ist es notwendig, in einer leicht gebeugten Stellung ebenfalls einen erhöhten Widerstand bereitzustellen. Dass das Gelenk in einer leicht gebeugten Stellung nicht vollständig sperrt, hat den Vorteil, dass der Nutzer des Gelenks noch Eingriffsmöglichkeiten in die Gelenksbewegung hat. Sollte er z.B. auf einer Treppe stehen und das Gleichgewicht verlieren, würde er dann über ein gesperrtes Gelenk unkontrolliert stürzen, während er ein Gelenk mit einem hohen Flexionswiderstand mittels der Stumpfkraft noch beugen und damit die Folgen des Sturzes vermindern oder das Stürzen ganz verhindern kann. Ebenfalls erleichtert eine hohe Dämpfung während des Stehens das Manövrieren des Gelenkes in engen Räumen oder das Niedersetzen.

Wenn das Gelenk einen hohen Widerstand bietet, auch wenn es nicht vollständig blockiert, ist das Radfahren nicht oder nur unter großen Anstrengungen möglich, weil nicht nur der Widerstand der Pedale, sondern auch der Widerstand des Gelenkes überwunden werden muss.

Aus der EP 549 855 A1 ist ein Verfahren bekannt, bei dem ein gesonderter Modus eingestellt werden kann, um den Beuge- und/oder Streckwiderstand zu reduzieren. Dadurch ist es möglich, Rad zu fahren. Das Gelenk bietet dann jedoch keine Sicherheit beim Stehen, beispielsweise wenn ein Prothesennutzer absteigt oder anhält und auf dem versorgten Bein steht. Um die gewünschte und erforderliche Sicherheit zu erhalten, muss erst wieder aktiv in den Normalmodus gewechselt werden. Dies geschieht beispielsweise durch eine Abfolge normalerweise nicht auftretender Bewegungsabfolgen, über die erkannt wird, dass ein Umschalten erfolgen soll. Dazu ist in der Regel notwendig, dass der Nutzer von dem Rad absteigt, so dass beim Umschalten in den Normalmodus eine potentiell unsichere Situation vorliegt.

Aufgabe der vorliegenden Erfindung ist es, ein Verfahren bereitzustellen, mit denen es möglich ist, die Widerstandseinrichtung des Gelenks, insbesondere des Knies, automatisch an das Radfahren anzupassen, ohne dass eine bewusste Aktivierung oder Deaktivierung des Modus erfolgen muss.

Erfindungsgemäß wird diese Aufgabe mit einem Verfahren gemäß dem Hauptanspruch erreicht. Vorteilhafte Ausgestaltungen und Weiterbildungen der Erfindung sind in den Unteransprüchen aufgeführt.

Das erfindungsgemäße Verfahren zur Steuerung eines künstlichen orthetischen oder prothetischen Gelenkes einer unteren Extremität mit einer Widerstandseinichtung, der zumindest ein Aktuator zugeordnet ist, über den der Beuge- und/oder Streckwiderstand in Abhängigkeit von Sensordaten verändert wird, wobei während der Benutzung des Gelenkes über Sensoren Zustandsinformationen bereitgestellt werden, sieht vor, dass eine vom Gehen abweichende zyklische Bewegung ermittelt und der Widerstand für die Dauer der zyklischen Bewegung reduziert wird. Dadurch ist es möglich, automatisch das Vorliegen eines besonderen, vom Gehen abweichenden Bewegungszustandes zu erkennen, zum Beispiel das Radfahren, um dann automatisch eine Anpassung des Widerstandes an das aktuelle Bewegungsverhalten zu ermöglichen. Durch das Ermitteln und Erkennen einer zyklischen Bewegung, wie sie das Radfahren darstellt, ist es möglich, einem Nutzer der Orthese oder Prothese jederzeit das Radfahren zu ermöglichen, ohne dass eine bewusste und in der Regel umständliche Aktivierung eines Sondermodus erfolgen muss. Die Reduzierung des Widerstandes erfolgt automatisch und wird für die Dauer der zyklischen, vom Gehen abweichenden Bewegung beibehalten. Wird die zyklische Bewegung beendet, beispielsweise wenn der Nutzer der Prothese oder Orthese anhält, oder absteigt, wird ebenfalls automatisch erkannt, dass die zyklische Bewegung beendet ist, so dass die Reduzierung des Widerstandes aufgehoben wird. Dadurch ist es möglich, dass eine ausreichende Sicherheit beim Anhalten oder Absteigen beim Fahrradfahren gewährleistet ist.

Die zyklische Bewegung wird bevorzugt über eine Auswertung kinematischer Größen ermittelt, insbesondere durch eine Auswertung von Gelenkwinkeldaten. Je nach Änderung des Gelenkwinkels kann erkannt werden, um welche Bewegungsart es sich handelt, wobei die zyklische Bewegung bevorzugt durch eine Auswertung der Phasenverschiebung zwischen einem Gelenkwinkel und einem Gelenkteilinertialwinkel oder zwei Gelenkteilinertialwinkeln oder der Änderung dieser Winkel ermittelt wird. Eine Inertialwinkelgeschwindigkeit ist diejenige Winkelgeschwindigkeit, die ein Gelenkteil gegenüber einer Orientierung ausübt, die nicht Teil des Gelenkes ist, vorzugsweise gegenüber der Schwerkraftorientierung. Das Radfahren kann also anhand eines zyklischen Verlaufes eines Gelenkwinkels, der Gelenkwinkelgeschwindigkeit, anhand der Phasenverschiebung zwischen dem Gelenkwinkel und einem Inertialwinkel eines Gelenkteils oder an der Phasenverschiebung zwischen der Gelenkwinkelgeschwindigkeit und der Inertialwinkelgeschwindigkeit eines Gelenkteils erfasst werden. Die Bewertung kann zum Beispiel anhand der Länge des Vektors bestimmt werden, der aus den Komponenten Kniewinkelgeschwindigkeit und Inertialwinkelgeschwindigkeit des Unterschenkelteils gebildet wird.

Weiterhin kann die zyklische Bewegung anhand des zeitlichen Abstandes von Maxima und Minima des Gelenkwinkels erkannt werden. Da es sich beim Radfahren um eine harmonische, sehr gleichmäßige Bewegung handelt, insbesondere hinsichtlich des Kniegelenkwinkels, können auch das Vorliegen oder Nichtvorliegen relativer Maxima oder Minima in dem Verlauf des Gelenkwinkels herangezogen werden, um das Radfahren zu erkennen. Während sich beim Gehen in der Ebene charakteristische relative Minima und Maxima in einem Bewegungszyklus ergeben, liegen andere charakteristische Minima und Maxima beim Radfahren in höherer Frequenz vor. Ebenfalls ist in der Regel davon auszugehen, dass beim Radfahren der Nutzer der Prothese oder Orthese das Kniegelenk nicht in die vollständige Streckung bringt, um ein Blockieren des Gelenks aufgrund des Prothesenaufbaues zu verhindern. Daher wird bei einem Nichtvorliegen einer vollständigen Streckung über mehrere Bewegungszyklen davon auszugehen sein, dass die Radfahrbewegung ausgeführt wird, so dass bei fortdauernder Bewegung der Widerstand reduziert wird. Es werden also für die auftretenden Maxima der Parameter, also des Gelenkwinkels, der Gelenkwinkeländerung oder dem Inertialwinkel Wertebereiche festgelegt, innerhalb derer sich die ermittelten Werte befinden müssen, damit eine zyklische Bewegung als gegeben ermittelt wird.

Ergänzend kann eine in einem unteren Anschlussteil wirksame Belastung, z.B. die Axialkraft ermittelt und der Widerstand bei Erreichen oder Unterschreiten eines Schwellwertes der Belastung reduziert werden. Zumindest bei der Aufwärtsbewegung werden keine oder nur sehr geringe Belastungen, beispielsweise Axialkräfte, auf einen Unterschenkel einwirken. Werden beispielsweise für zwei Umdrehungen jeweils zyklisch abnehmende Axialkraftverläufe ermittelt, kann daraus geschlossen werden, dass der Nutzer gerade Rad fährt, so dass eine Reduzierung des Widerstandes oder der Widerstände angezeigt ist.

Da der Prothesennutzer oder Orthesennutzer beim Radfahren stets eine Beugung des Kniegelenkes vorsieht, um ein Blockieren aufgrund des mechanischen Aufbaus des Gelenkes zu vermeiden, kann als weitere Bedingung vorgesehen sein, dass ein minimaler Gelenkwinkel vorhanden sein muss, damit der Widerstand reduziert wird. Dadurch wird gewährleistet, dass bei einem gestreckten Kniegelenk oder bei einem nahezu gestreckten Kniegelenk stets ein erhöhter Widerstand bereitgestellt wird, da aus dieser Stellung geschlossen werden kann, dass das Radfahren aktuell beendet oder unterbrochen wird.

Aus Sicherheitsgründen ist vorgesehen, dass eine Widerstandserhöhung erfolgt und somit in einen Normalmodus zurückgeschaltet wird, wenn die Bedingungen für eine Reduzierung des Widerstandes nicht mehr vorliegen. Als weitere Sicherheitseinrichtung kann vorgesehen werden, dass der Flexionswiderstand stets während der Extensionsbewegung erhöht wird, also dass der Flexionswiderstand beim Heruntertreten der Pedale erhöht wird, da diese Widerstandserhöhung sich nicht negativ auf das Radfahren auswirkt, jedoch bei einem spontanen Absteigen, Anhalten oder einer Abstützbewegung ein unkontrolliertes Einknicken des Gelenkes des versorgten Beines verhindert wird.

Eine Vorrichtung soll zur Durchführung des oben beschriebenen Verfahrens mit einer einstellbaren Widerstandseinrichtung, die zwischen zwei gelenkig aneinander gelagerten Komponenten eines künstlichen orthetischen oder prothetischen Gelenkes einer unteren Extremität angeordnet ist, mit einer Steuereinrichtung und Sensoren, die Zustandsinformationen der Vorrichtung erfassen, sieht vor, dass eine Einstellvorrichtung vorgesehen ist, und dass über die Einstellvorrichtung eine Bewegungsmusterbedingte Widerstandsänderung aktivierbar und/oder deaktivierbar ist. Neben einer automatischen Aktivierung kann somit auch eine bewusste Aktivierung des Radfahrmodus erfolgen. Ebenfalls ist es möglich, dass die automatische Erkennung ausgeschaltet wird, wenn dieser Modus nicht benötigt wird.

Eine solche Funktion kann die einzige Steuerungsfunktion einer Steuerung sein. Ebenfalls ist es möglich, dass sie nur Teil einer funktionalen Steuerung ist, um als ein Zusatzmodus bereitzustehen, der während des Gehprogrammes zu jeder Zeit, in der die Bedingungen erfüllt sind, den Widerstand des Gelenkes reduziert.

Nachfolgend wird ein Ausführungsbeispiel der Erfindung anhand der beigefügten Figuren näher erläutert. Es zeigen:
- Figur 1 -: eine schematische Darstellung einer Prothese;
- Figur 2 -: den Kniewinkelverlauf über die Zeit beim Radfahren; sowie
- Figur 3: eine Konvention der Winkelangaben.

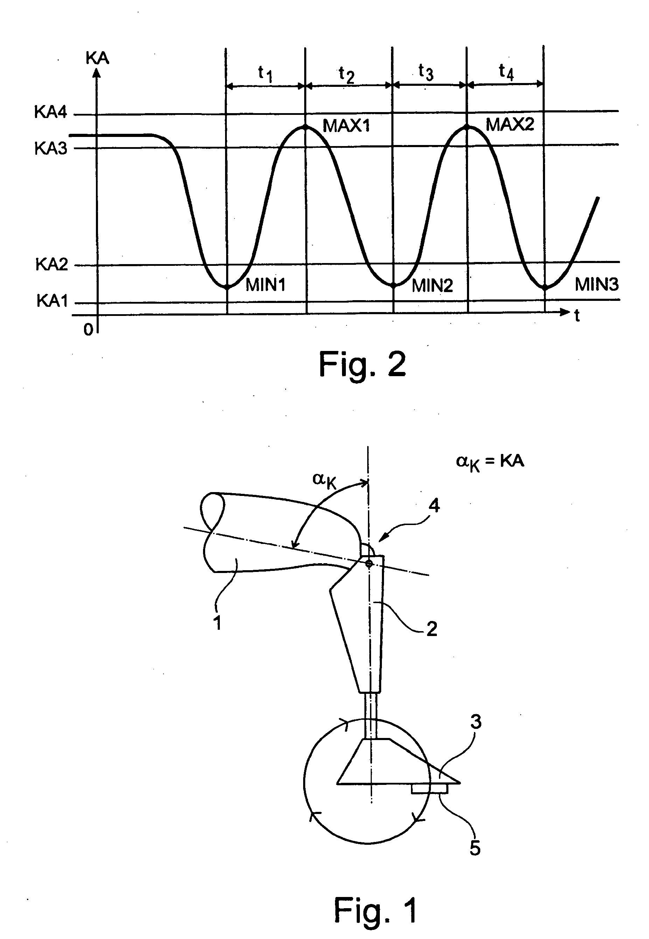

In der Figur 1 ist eine Prothese mit einem Oberschenkelschaft 1, einem gelenkig an dem Oberschenkelschaft 1 befestigten Unterschenkelteil 2 und einem Prothesenfuß 3 dargestellt. Der Oberschenkelschaft 1 ist über ein Prothesenkniegelenk 4 mit dem Unterschenkelteil 2 gekoppelt. In dem Oberschenkelschaft 1 wird der Oberschenkel eines Prothesennutzers aufgenommen, in dem Unterschenkelteil 2 sind und Widerstandseinrichtungen vorgesehen, um den Widerstand der Verschwenkbewegung zwischen dem Oberschenkelschaft 1 und dem Unterschenkelteil 2 einstellen zu können. In dem dargestellten Ausführungsbeispiel befindet sich der Prothesenfuß 3 auf einer Pedale 5, die kontralaterale, nicht versorgte Seite ist nicht dargestellt. Innerhalb des Unterschenkelteils 2 ist eine Steuerungseinrichtung angeordnet, die mit Sensoren gekoppelt ist. Diese Sensoren erfassen Zustandsdaten der Prothese, insbesondere Kräfte, Momente, Winkelgeschwindigkeiten und Winkel. Auf Grundlage der Sensordaten wird in der Steuereinrichtung ermittelt, welche Bewegung gegenwärtig vorliegt, so dass automatisch der richtige Widerstand in der Widerstandseinrichtung eingestellt wird, um die Bewegung korrekt und sicher auszuführen.

Im vorliegenden Fall soll der Zustand des Radfahrens ermittelt werden. Fahrradfahren ist durch eine zyklische Bewegung gekennzeichnet, weil die Pedale 5 und damit auch der auf der Pedale 5 aufgesetzte Prothesenfuß 3 eine kreisförmige Bewegung in einer im Wesentlichen gleich bleibenden Drehbewegungsrichtung ausführt. Dies ist durch den Pfeil in der Figur 1 angedeutet. Der Oberschenkelschaft 1 führt eine Schwenkbewegung um das Hüftgelenk aus, während das Unterschenkelteil 2 eine Rotationsbewegung um die Gelenkachse des Prothesenkniegelenkes 4 und eine auf- und abgehende Translationsbewegung durchführt.

Der Kniewinkel α_{K} wird zwischen der Längsachse des Unterschenkelteils 2 und der Längsachse des Oberschenkelschaftes 1 gemessen, wobei sich die beiden Längsachsen im Prothesenkniegelenk 1 schneiden. Grundsätzlich sind auch andere Bezugsachsen möglich.

Der Verlauf des Kniewinkels KA ist in der Figur 2 über die Zeit dargestellt. Ausgehend von einer maximal gebeugten Stellung zum Zeitpunkt t=0 wird bei Beginn des Radfahrens zunächst das versorgte Bein heruntergedrückt, so dass sich ein erstes Kniewinkelminimum MIN1 ergibt. Anschließend verläuft über den Zeitraum t₁ der Kniewinkel im Wesentlichen sinusartig bis zum ersten Kniewinkelmaximum MAX1 und von dort aus wieder sinusförmig zum zweiten Kniewinkelminimum MIN2. Dieses Bewegungsmuster und der Kniewinkelverlauf setzen sich dann entsprechend fort, so dass sich eine ungefähr sinusförmige Kniewinkelkurve ergibt.

Charakteristisch an dem Kniewinkelverlauf beim Fahrradfahren ist die Tatsache, dass die Prothese nie vollständig gestreckt ist, so dass sich das Kniewinkelminimum zwischen dem nicht ganz gestreckten Bein mit dem Kniewinkel KA1 und dem etwas weiter gebeugten Bein KA2 befindet, während sich das Kniewinkelmaximum zwischen den Werten KA3 und KA4 befindet, wobei diese Werte von der Sattelhöhe und der Pedallänge abhängen.

Wird in der Steuereinrichtung aufgrund der Sensordaten ein zyklischer Verlauf der Bewegung erkannt, so dass die Minima und Maxima innerhalb des unteren und oberen Kniewinkelbereichs im Wesentlichen zeitlich gleich verteilt sind und über einen längeren Zeitraum andauern, wird die Widerstandseinrichtung so eingestellt, dass ein verringerter Widerstand bereitgestellt wird. Dabei können sowohl der Beuge- als auch der Streckwiderstand verringert werden, um dem Rad fahrenden Prothesenträger nicht auch den Extensions- und Flexionswidertand der Widerstandseinrichtung überwinden lassen zu müssen.

Es ist vorgesehen, dass die ersten Bewegungszyklen noch mit einem erhöhten Widerstand durchgeführt werden, um eine erhöhte Sicherheit für den Prothesenträger bereitzustellen. Erst nach einer gewissen Anzahl an Umdrehungen der Pedale 5 wird dann der Widerstand verringert. Die Anzahl der notwendigen Umdrehungen kann eingestellt werden, beispielsweise zwischen zwei und fünf Umdrehungen.

Ergänzend oder alternativ zu der Auswertung der zyklischen Bewegung auf Grundlage des Kniewinkels KA können andere kinematische Größen verwendet werden, zum Beispiel Beschleunigungen oder Geschwindigkeiten, die in einem wiederkehrenden Muster beim Fahrradfahren auftreten, beispielsweise die spezielle Kreisbewegung des Prothesenfußes 3 oder die zyklische Verschwenkbewegung in Verbindung mit der auf- und abgehenden Bewegung des Unterschenkelteils 2. Diese kinematischen Größen können entweder als Alternative oder ergänzend zu dem Kniewinkel KA zur Ermittlung der zyklischen Bewegung herangezogen werden.

Ebenfalls können Kräfte oder Momente zur Ermittlung der zyklischen Bewegung herangezogen werden, beispielsweise eine axiale Kraft, die in der Längsrichtung des Unterschenkelteils 2 wirkt oder auf den Prothesenfuß 3 ausgeübt wird, um ein Vorliegen einer Radfahrbewegung zu ermitteln. Die Belastung, z.B. die Axialkraft, ist auch als ein statisches Kriterium vorgesehen. Liegt eine bestimmte Belastung vor, wird nicht auf die Situation des Radfahrens geschlossen, unterschreitet die Belastung eine Schwellwert, kann in Verbindung mit anderen Daten auf eine Radfahrsituation geschlossen werden, so dass eine angepasste Widerstandseinstellung erfolgen kann.

Als Sicherheitseinrichtung ist vorgesehen, dass eine Flexionswiderstandserhöhung während der Extensionsbewegung erfolgt, also wenn die Prothese gestreckt wird. Dies dient, zur Erhöhung der Sicherheit, da der erhöhte Flexionswiderstand bei der Extensionsbewegung sich nicht negativ auf das Radfahren auswirkt, jedoch bei einem unbeabsichtigten Absteigen oder plötzlichen Anhalten eine Sicherheit gegen ein unbeabsichtigtes Einbeugen der Prothese gewährleistet.

Als weitere Sicherheitseinrichtung ist vorgesehen, dass der reduzierte Widerstand, der auf Null reduziert werden kann, auf den Normalwert erhöht wird, wenn die zyklischen Bewegungen aufhören, um so automatisch bei einem Anhalten und Absteigen in den Normalmodus zu schalten, das heißt, dass unmittelbar nach dem Absteigen die Gehfunktion bereitstellt wird.

Befindet sich das Bein in einer gestreckten oder nahezu gestreckten Stellung, wird der Widerstand ebenfalls erhöht bzw. nicht verringert, um zu verhindern, dass bei einem gestreckten Bein in einer stehenden Stellung bei Vorliegen zyklischer Belastungen keine Sicherheit vorhanden ist.

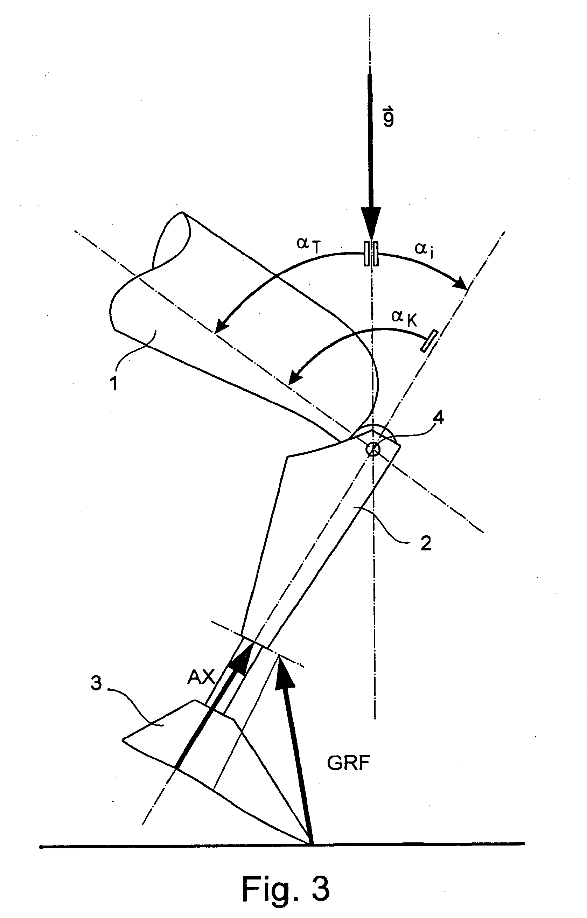

In der Figur 3 ist schematisch eine Prothese in einer angewinkelten Position dargestellt. Um die Widerstandsveränderungen und eine Zustandsermittlung automatisch durchführen zu können, ist es vorgesehen, dass der Inertialwinkel α_{T} und/oder der Kniewinkel α_{K} gemessen werden. Der Inertialwinkel α_{T} des Oberschenkelteils 1 wird zur Vertikalen gemessen, die in Schwerkraftrichtung wirkend angenommen wird. In der Figur 3 ist dies durch den Schwerkraftvektor g angedeutet. Als Bezugsgröße für den Inertialwinkel α_{T} wird die Längsachse des Oberschenkelteils 1 angenommen, die durch die Schwenkachse des Prothesenkniegelenkes 4 geht. Dabei entspricht die Längsachse ungefähr der Orientierung eines natürlichen Oberschenkelknochens und erstreckt sich im Wesentlichen mittig zu dem Oberschenkelteil 1, das in der Regel als ein Oberschenkelschaft ausgebildet ist.

Der Kniewinkel α_{K} liegt zwischen der Längsstreckung des Unterschenkelteils 2 und der Längserstreckung des Oberschenkelteils 1 an. Auch hier geht die Längserstreckung des Unterschenkelteils 2 durch die Gelenkachse des Prothesenkniegelenkes 4. Der Kniewinkel α_{K} kann sich aus dem Inertialwinkel α_{T} des Oberschenkelteils 1 und dem Inertialwinkel αᵢ des Unterschenkelteils 2 errechnen lassen, wobei aufgrund der Berechnung der Inertialwinkel α_{T} und αᵢ ausgehend von dem Schwerkraftvektor g eine angepasste Vorzeichenregelung eingeführt wird, so dass der Inertialwinkel α_{T} des Oberschenkelteils 1 sich aus der Differenz zwischen dem Kniewinkel α_{K} und dem Inertialwinkel αᵢ des Unterschenketeils 2 ergibt.

Darüber wird die Bodenreaktionskraft GRF bzw. die Axialkraft AX, die in Längsrichtung des Unterschenkelteils 2 wirksam ist, bestimmt, um auf Grundlage der vorliegenden Kräfte zu entscheiden, in welchem Bewegungszustand sich der Prothesennutzer befindet.

## Patentansprüche

1. Verfahren zur Steuerung eines künstlichen orthetischen oder prothetischen Gelenkes (4) einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes (4) über Sensoren Zustandsinformationen bereitgestellt werden, **dadurch gekennzeichnet, dass** eine vom Gehen abweichende zyklische Bewegung ermittelt und der Widerstand für die Dauer der zyklischen Bewegung reduziert wird.

2. Verfahren nach Anspruch 1, **dadurch gekennzeichnet, dass** die zyklische Bewegung durch eine Auswertung kinematischer Größen ermittelt wird.

3. Verfahren nach Anspruch 1 oder 2, **dadurch gekennzeichnet, dass** die zyklische Bewegung durch eine Auswertung der Phasenverschiebung zwischen einem Gelenkwinkel (α_{K}, KA) und einem Gelenkteilinertialwinkel (α_{T}, αᵢ) oder zwei Gelenkteilinertialwinkeln (α_{T}, αᵢ) oder der Änderung dieser Winkel (α_{T}, αᵢ, α_{K}, KA) ermittelt wird.

4. Verfahren nach einem der voranstehenden Ansprüche, **dadurch gekennzeichnet, dass** die zyklische Bewegung anhand des zeitlichen Abstandes von Maxima und Minima des Gelenkwinkels (α_{K}, KA) oder Gelenkteilinertialwinkels (α_{T}, αᵢ) ermittelt wird.

5. Verfahren nach Anspruch 4, **dadurch gekennzeichnet, dass** für die Maxima und Minima Wertebereiche festgelegt werden, innerhalb derer sich die ermittelten Werte befinden müssen, damit eine zyklische Bewegung als gegeben ermittelt wird.

6. Verfahren nach einem der voranstehenden Ansprüche, **dadurch gekennzeichnet, dass** eine in einem unteren Anschlussteil (2, 3) wirksame Belastung ermittelt und der Widerstand bei Erreichen oder Unterschreiten eines Schwellwertes der Belastung reduziert wird.

7. Verfahren nach einem der voranstehenden Ansprüche, **dadurch gekennzeichnet, dass** ein minimaler Gelenkwinkel (α_{K}, KA) festgelegt wird, der vorhanden sein muss, damit der Widerstand reduziert wird.

8. Verfahren nach einem der voranstehenden Ansprüche, **dadurch gekennzeichnet, dass** eine Widerstandserhöhung erfolgt, wenn die Bedingungen für eine Reduzierung des Widerstandes nicht mehr vorliegen.

9. Verfahren nach einem der voranstehenden Ansprüche, **dadurch gekennzeichnet, dass** der Flexionswiderstand während der Extensionsbewegung erhöht wird.

10. Verfahren nach einem der voranstehenden Ansprüche, **dadurch gekennzeichnet, dass** mehrere Steueralgorithmen vorhanden sind, die auf der Grundlage von Messwerten unterschiedlicher Einrichtungen zur Erfassung von Winkeln und Kräften arbeiten, so dass bei Ausfall einer Einrichtung zur Erfassung von Winkeln und Kräften die übrigen Messwerte zur Steuerung der Veränderung des Streck- und/oder Beugewiderstandes verwendet werden.

## Claims

1. A method for controlling an artificial orthotic or prosthetic joint of a lower, extremity (4) with a resistance device to which at least one actuator is assigned, via which actuator the flexion and/or extension resistance is changed depending on sensor data, with status information being made available via sensors during the use of the joint, **characterized in that** a cyclic movement different from walking is determined, and the resistance is reduced for the duration of the cyclic movement.

2. The method as claimed in claim 1, **characterized in that** the cyclic movement is determined by an evaluation of kinematic variables.

3. The method as claimed in claim 1 or 2, **characterized in that** the cyclic movement is determined by an evaluation of the phase shift between a joint angle (a_{K}, KA) and a joint-part inertial angle (α_{T}, αᵢ) or two joint-part inertial angles (α_{T}, αᵢ, α_{K}, KA) or the change of these angles.

4. The method as claimed in one of the preceding claims, **characterized in that** the cyclic movement is determined on the basis of the time interval between maxima and minima of the joint angle (α_{K}, KA) or joint-part inertial angle (α_{T}, αᵢ).

5. The method as claimed in claim 4, **characterized in that**, for the maxima and minima, value ranges are established within which the determined values must be situated for a cyclic movement to be determined as given.

6. The method as claimed in one of the preceding claims, **characterized in that** a load acting in a lower attachment part (2, 3) is determined, and the resistance is reduced when the load reaches or is below a threshold value.

7. The method as claimed in one of the preceding claims, **characterized in that** a minimal joint angle (α_{K}, KA) is established that has to be present for the resistance to be reduced.

8. The method as claimed in one of the preceding claims, **characterized in that** the resistance is increased if the conditions for reducing the resistance are no longer present.

9. The method as claimed in one of the preceding claims, **characterized in that** the flexion resistance is increased during the extension movement.

10. The method as claimed in one of the preceding claims, **characterized in that** a plurality of control algorithms are present which, on the basis of measured values of different devices for detecting angles and forces, operate in such a way that, if a device for detecting angles and forces fails, the other measured values are used to control the change in the extension and/or flexion resistance.

## Revendications

1. Procédé pour commander une articulation orthétique ou prothétique artificielle (4) d'une extrémité inférieure avec un système à résistance auquel est associé au moins un actionneur au moyen duquel on modifie la résistance à la flexion et/ou à l'extension en fonction de données sensorielles, dans lequel on met à disposition des informations d'état via des capteurs pendant l'utilisation de l'articulation (4),
**caractérisé en ce que** l'on détecte un mouvement cyclique s'écartant de la marche et la résistance est réduite pendant la durée du mouvement cyclique.

2. Procédé selon la revendication 1, **caractérisé en ce que** le mouvement cyclique est détecté par une analyse de grandeurs cinématiques.

3. Procédé selon la revendication 1 ou 2, **caractérisé en ce que** le mouvement cyclique est détecté par une analyse du décalage de phase entre un angle d'articulation (α_{K}, KA) et un angle d'inertie d'une partie d'articulation (α_{T}, αᵢ) ou entre deux angles d'inertie de parties d'articulation (α_{T}, αᵢ), ou de la variation de cet angle (α_{T}, αᵢ, α_{K}, KA).

4. Procédé selon l'une des revendications précédentes, **caractérisé en ce que** le mouvement cyclique est détecté au moyen de la distance temporelle des maxima et des minima de l'angle d'articulation (α_{K}, KA) ou de l'angle d'inertie d'une partie d'articulation (α_{T}, αᵢ).

5. Procédé selon la revendication 4, **caractérisé en ce que** l'on fixe pour les maxima et les minima des plages de valeurs à l'intérieur desquelles les valeurs détectées doivent se trouver afin de détecter qu'il existe un mouvement cyclique.

6. Procédé selon l'une des revendications précédentes, **caractérisé en ce que** l'on détecte une charge agissant dans une pièce de raccordement inférieure (2, 3), et **en ce que** l'on réduit la résistance lorsqu'on atteint ou lorsqu'on passe au-dessous d'une valeur seuil de la charge.

7. Procédé selon l'une des revendications précédentes, **caractérisé en ce que** l'on fixe un angle d'articulation minimum (α_{K}, KA), qui doit se présenter afin de réduire la résistance.

8. Procédé selon l'une des revendications précédentes, **caractérisé en ce que** l'on procède à une augmentation de résistance quand les conditions pour une réduction de la résistance ne se présentent plus.

9. Procédé selon l'une des revendications précédentes, **caractérisé en ce que** la résistance à la flexion est augmentée pendant le mouvement d'extension.

10. Procédé selon l'une des revendications précédentes, **caractérisé en ce qu'**il comprend plusieurs algorithmes de commande qui fonctionnent en se basant sur des valeurs de mesures de systèmes différents pour la détection d'angles et de forces, de sorte qu'en cas de défaillance d'un système pour la détection d'angles et de forces on utilise les autres valeurs de mesures pour la commande de la modification de la résistance à l'extension et/ou à la flexion.
